# EUROPEAN PATENT SPECIFICATION

(11) **EP 1 683 416 B1**
(45) Date of publication and mention of the grant of the patent: **09.12.2015**
(21) Application number: 06100226.7
(22) Date of filing: 11.01.2006
(51) Int. Cl.: A01N 43/40, A01P 1/00, A61K 8/49, A61K 8/33, A61K 8/34, A61Q 19/10

(54) **Alcoholic compositions for disinfection**
Alkoholische Zusammensetzungen zum Desinfizieren
Compositions alcooliques pour la désinfection

(30) Priority: 19.01.2005 DE 102005002645
(43) Date of publication of application: 26.07.2006
(60) Divisional application: 10179983.1
(73) Proprietor: Air Liquide Santé (International), 75341 Paris Cedex 07 (FR); Schuelke & Mayr, 22851 Norderstedt (DE)
(72) Inventor: Beilfuss, Wolfgang, 22339 Hamburg (DE); Behrends, Sabine, 25421 Appen (DE); Goroncy-Bermes, Peter, 22145 Hamburg (DE); Puchstein, Burghard, 20257 Hamburg (DE)
(74) Representative: Conan, Philippe Claude

(56) References cited:
- EP-A- 1 468 700
- WO-A-98/20095
- GB-A- 1 533 952
- US-A- 4 542 125

## Description

The present invention relates to alcoholic compositions and their use for disinfecting animate and inanimate surfaces, in particular for surgical and hygienic hand disinfection and disinfectant handwashing.

The aim of hand disinfection, hand decontamination and skin antisepsis is to prevent transmission of microorganisms and viruses or to suppress their unwanted introduction into endangered regions of the body or more sensitive regions. Compositions for hygienic hand disinfection and for disinfectant handwashing must satisfy certain efficacy requirements, some of which are defined in standards. Various methods are possible for treating the hands after contamination.

Hygienic hand disinfection complying with EN 1500 as rubbing method without addition of water causes the death or inactivation of the transient microorganisms on the hands, without any risk of microbes being disseminated in the surroundings and without any risk of recontamination of the hands by microorganisms possibly present in the water. Disinfectant handwashing complying with EN 1499 with a microbicidal composition using tap water is likewise directed against transient microorganisms without precluding their dissemination in the surroundings. It serves in particular to reduce microbes during the washing procedure, but cannot replace hand disinfection. In disinfectant handwashing, the composition is rubbed in undiluted and foamed with a little water, and the hands are cleaned and thoroughly rinsed with water.

Compositions for disinfectant handwashing and hygienic hand disinfection must be effective after acting for short times (e.g. 30 seconds or 1 minute). In the efficacy tests, it is important that there is a good effect (RF > 3 orders of magnitude) after acting for these short times. For toxicological reasons it is additionally necessary for, besides adequate efficacy, in particular the compatibility with human skin to be ensured, even if the intended use is on inanimate surfaces.

Commercially available compositions for disinfectant handwashing are usually alcohol- or surfactant-containing liquid soaps and wash lotions ready for use with further biocidal agents added. Known compositions comprise as biocidal agents for example short-chain alcohols and as excipients superfatting agents, moisturizers and fragrances to improve the skin compatibility and acceptance. To enhance the efficacy and to achieve a residual effect intended to prevent the number of microbes on the hands increasing again, the known compositions often also comprise additional agents such as biguanides (e.g. chlorhexidine), quaternary ammonium compounds (e.g. benzalkonium chloride), phenol derivatives (e.g. ortho-phenylphenol) or carboxylic acids. For example, a known alcoholic hand disinfection composition includes about 53% by weight propanols and about 0.8% by weight chlorhexidine digluconate, and excipients. A further known alcoholic hand disinfection composition includes approximately 75% by weight ethanol, 0.1% by weight 2-biphenylol, polyvidone 30 and excipients.

The known compositions for disinfectant handwashing have a number of disadvantages. Thus, some products show an efficacy which is not always satisfactory, or the desired efficacy is achieved only after acting for a lengthy time. Some compositions additionally have insufficient skin compatibility. Thus, compositions based on chlorhexidine are reported to be prone to skin incompatibility, it being suggested for example that there is partial release of chloroaniline.

An additional factor is that agents with organically bound halogen such as chlorhexidine have only conditional environmental compatibility. Chlorhexidine is moreover sufficiently effective only with a comparatively high concentration of the agent (e.g. 2% by weight) in wash products and may lead to discolorations on contaminated surfaces. Poly(hexamethylenebiguanide) hydrochloride is a polymeric biguanide salt whose structure is not exactly defined. For this reason, no medicinal product with this agent yet has marketing authorization in Germany. The polymeric biguanide may be employed only in a concentration of up to 0.3% by weight in cosmetic compositions.

Hence there is a need for compositions with improved microbicidal efficacy and, at the same time, good skin compatibility for use in disinfection.

British patent application GB 1533 952 A discloses bispyridiniumalkanes as antimicrobials, in soaps and pre-operative skin treatments, the formulation with alcohol being described as suitable.

United States patent US 4542125 A discloses octenidine in combination with alcohol such as isopropanol and also in combination with quaternary ammonium compounds for disinfecting skin.

European patent application EP 1 468 700 A discloses Sensiva™SC 50 as having intrinsically antimicrobial properties.

International application WO 98/20095 discloses octenidine in combination with C₁-C₈ alcohols and a surfactant for washing disinfection of hands.

It has now surprisingly been found that the problems of the prior art are solved by alcoholic compositions according to Claim 1.

Compositions preferred in this connection are those free of aromatic alcohols such as 2-phenoxyethanol, benzyl alcohol, phenethyl alcohol, 1-phenoxypropan-2-ol, 3-(4-chlorophenoxy)-1,2-propanediol, chlorobutanol, 2,4-dichlorobenzyl alcohol or mixtures thereof. Preferred compositions are moreover those including no salts selected from the salts of benzoic acid, propionic acid, salicylic acid, sorbic acid, 4-hydroxy-benzoic acid, dehydroacetic acid and 10-undecylenic acid and/or any of the free acids mentioned.

### a) Glycerol monoalkyl ethers

According to the invention glycerol monoalkyl ethers are selected from 1-(2-Ethylhexyl)glycerol ether (Sensiva^{®} SC 50) and 1-dodecylglycerol ether. In a further embodiment 3-(1-menthoxy)-1,2-propanediol (glycerol monomenthyl ether) is employed. Glycerol monomenthyl ether reduces a possible burning sensation of the alcoholic compositions according to the invention on (damaged) skin.

The content of glycerol monoalkyl ether is generally in the range from 0.04 to 1% by weight, preferably 0.08 to 1% by weight, such as 0.15 to 0.5% by weight, for example about 0.25% by weight.

### b) Bispyridiniumalkane

According to the invention, the bis[4-(substituted amino)-1-pyridinium]alkane of the general formula I or II: 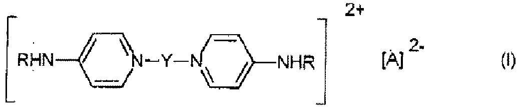 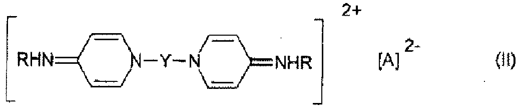 where Y is an alkylene group having 4 to 18 carbon atoms, R is an alkyl group having 6 to 18 carbon atoms or a cycloalkyl group having 5 to 7 carbon atoms or the phenyl radical which may be substituted by a halogen atom, and A is an anion or a plurality of anions employed according to the invention is N,N'-(1,10-decanediyldi-1[4H]-pyridinyl-4-ylidene)bis(1-octanamine) dihydrochloride (octenidine dihydro-chloride). Octenidine disulphate is also suitable moreover.

In a preferred embodiment, the content of bispyridiniumalkane is in the range from 0.01 to 1% by weight, in particular 0.02 to 0.5% by weight, such as 0.05 to 0.2% by weight, for example about 0.1% by weight.

### c) Aliphatic alcohols

Suitable aliphatic alcohols employed according to the invention, are selected from ethanol, isopropanol and n-propanol as well as mixtures thereof, ethanol being particularly preferred in all embodiments of the invention.

The content of aliphatic alcohol in the compositions according to the invention is preferably in the range from 50 to 95% by weight.

The amount of quaternary ammonium salt (is preferably from 0.02 to 2.5% by weight, in particular 0.04 to 0.8% by weight, such as 0.05 to 0.4% by weight.

Moreover, compositions which are preferred also include 1 to 5% by weight, preferably 2 to 3% by weight of superfatting agents, such as polyol fatty acid esters, e.g. glycerol esters, preferably glycerol cocoate, isopropyl myristate, isopropyl palmitate and triglycerides.

Compositions which are additionally preferred are those additionally including from 0.05 to 0.8% by weight, more preferably 0.1 to 0.5% by weight of skin care additives such as allantoin, glycerol or sodium gluconate.

The compositions of the invention are preferably present in aqueous form and comprise from 1 to 80% by weight, more preferably 2 to 65% by weight, in particular 5 to 50% by weight, such as 8 to 40% by weight, 10 to 35% by weight, 12 to 30% by weight or 15 to 22% by weight of water, based on the composition. In a preferred embodiment the water content of the compositions according to the invention is less than 55% by weight, more preferably less than 50% by weight, in particular less than 40% by weight.

The composition may additionally comprise functional additives such as colorants, perfume, buffers, electrolytes and moisturizing factors. The preferred pH of the composition is from 4 to 8.

The invention further relates to the use of the abovementioned compositions for disinfection or decontamination of animate (e.g. skin, hands, mucous membrane, wounds) and inanimate surfaces (e.g. apparatuses, instruments, endoscopes). The compositions employed as products for personal hygiene and as medical wash products, high-value, soap-free wash products for all hand, skin and body washing and as bath additive.

The compositions according to the invention are used in particular for hygienic hand disinfection or for disinfectant handwashing, in particular as skin antiseptic. The compositions according to the invention are suitable for hygienic and surgical hand disinfection, hygienic handwashing, hand decontamination, skin decontamination, personal hygiene washing lotion, as antimicrobial washing lotion, for (whole) body washing and care in connection with MRS (methicillin-resistant Staphylococcus aureus) and furthermore for disinfectant handwashing, for hygienic catheter care in patients, as handwashing product such as, for example, as antimicrobial soap, handwashing gel or handwashing lotion. The compositions can be used advantageously in all sectors with enhanced hygiene requirements in the medical and nonmedical sector, e.g. hospitals, medical practices, old people's and nursing homes, and in the food products and kitchen sectors. The invention relates in a particularly preferred embodiment to the non-therapeutic use of the compositions for surgical and hygenic hand disinfection and disinfectant handwashing.

The compositions can additionally also be formulated as gel compositions, ointment compositions and antimicrobial coatings and have an excellent effect with, at the same time, good skin compatibility and stability. The compositions show a distinctly improved efficacy compared with known compositions. Synergistic increases in effect are observed here in some cases. The possible inhibition of the efficacy of bispyridiniumalkanes, which is observed with many surfactants, does not occur with aliphatic alcohols. The excellent skin compatibility of the compositions is also to be emphasized.

It was surprising that a disinfectant effect is achieved after only a short time with alcoholic compositions according to the invention containing glycerol monoalkyl ether. Although glycerol monoalkyl ethers are known, for example from DE 42 40 674 C1, to act as deodorant agents, disinfection is immaterial for deodorants, since known deodorant agents should merely have antimicrobial activity, whereas hand disinfectants must have microbicidal activity. An additional factor is that glycerol monoalkyl ethers on their own have virtually no microbicidal effect, and accordingly are unsuitable as (sole) agents for hand disinfection. It was therefore surprising that glycerol monoalkyl ethers contribute in the compositions according to the invention to the disinfectant effect.

In addition, modern deodorant compositions are preferably free of relatively large amounts of aliphatic alcohols. Thus, skin compatibility of deodorant agents in combination with relatively large amounts of aliphatic alcohols is immaterial in deodorant compositions. By contrast, it was surprising that compositions according to the invention can be formulated with large amounts of aliphatic alcohol in some cases, without the efficacy of the bispyridiniumalkanes and glycerol monoalkyl ethers being impaired, and without skin incompatibility being observed.

The compositions according to the invention additionally have the following advantages:
- Good tolerability in combination with good antimicrobial efficacy and excellent short-term effect (acting for a time of, for example, 30 or 60 seconds), and very good effect on Gram-negative bacteria.
- Good efficacy for viruses (enveloped and non-enveloped) and multidrug-resistant microorganisms such as MRSA.

- No need to add a further preservative because they are self-preservative.
- The solubility in water and water-based compositions of glycerol monoalkyl ethers (such as 1-(2-ethylhexyl)glycerol ether) is only limited (solubility in water 0.1% by weight). The presence of bispyridiniumalkane in the compositions according to the invention leads to a significantly improved solubility in water of the glycerol monoalkyl ether.

The good efficacy of the compositions according to the invention can be verified in the quantitative suspension test. Compare standard methods of the DGHM for testing chemical disinfection methods, J. Gebel, H.-P. Werner, A. Kirsch-Altena, K. Bansemir, mhp Verlag GmbH, Wiesbaden, Germany, method 9.1 (date: 1 September 2001) (quantitative suspension test with bacteria (apart from mycobacteria and fungi)).

## Claims

1. Alcoholic composition which includes
a) from 0.02 to 2% by weight one or more 1- or 2-(C₁- to C₂₄-alkyl) glycerol ethers selected from 1-(2-Ethylhexyl) glycerol ether, 1-dodecylglycerol ether and 3-(1-menthoxy)-1,2-propanediol,
b) 0.01 to 2% by weight one or more bispyridiniumalkanes selected from (octenidine dihydrochloride) and Octenidine disulphate,
c) from 50 to 99% by weight one or more aliphatic alcohols selected from ethanol, isopropanol and n-propanol,
the composition also comprising from 0.01 to 5% by weight one or more quarternary ammonium compounds selected from mecetronium etilsulfate and benzalkonium chloride.

2. Composition according to Claim 1, **characterized in that** the glycerol monoalkyl ether is 1-(2-ethylhexyl)glycerol ether.

3. Composition according to any of the preceding claims, **characterized in that** it includes from 0.04 to 1% by weight by weight glycerol monoalkyl ether.

4. Composition according to any of the preceding claims, **characterized in that** the bispyridiniumalkane is octenidine dihydrochloride.

5. Composition according to any of the preceding claims, **characterized in that** it includes from 0.01 to 1% by weight bispyridiniumalkane.

6. Composition according to any of the preceding claims, **characterized in that** the aliphatic alcohol is ethanol.

7. Non-therapeutic use of a composition according to any of the preceding claims for hygienic hand disinfection or for disinfectant handwashing.

## Patentansprüche

1. Alkoholische Zusammensetzung, die Folgendes enthält:
a) 0,02 bis 2 Gew.-% eines oder mehrerer 1- oder 2-(C₁-C₂₄-Alkyl)glycerolether, ausgewählt aus 1-(2-Ethylhexyl)glycerolether, 1-Dodecylglycerolether und 3-(1-Menthoxy)-1,2-propandiol,
b) 0,01 bis 2 Gew.-% eines oder mehrerer Bispyridiniumalkane, ausgewählt aus Octenidindihydrochlorid und Octenidindisulfat,
c) 50 bis 99 Gew.-% eines oder mehrerer aliphatischer Alkohole, ausgewählt aus Ethanol, Isopropanol und n-Propanol,
wobei die Zusammensetzung ebenfalls 0,01 bis 5 Gew.-% einer oder mehrerer quaternärer Ammoniumverbindungen umfasst, ausgewählt aus Mecetroniumetilsulfat und Benzalkoniumchlorid.

2. Zusammensetzung nach Anspruch 1, **dadurch gekennzeichnet, dass** der Glycerolmonoalkylether 1-(2-Ethylhexyl)glycerolether ist.

3. Zusammensetzung nach einem der vorhergehenden Ansprüche, **dadurch gekennzeichnet, dass** sie 0,04 bis 1 Gew.-% Glycerolmonoalkylether enthält.

4. Zusammensetzung nach einem der vorhergehenden Ansprüche, **dadurch gekennzeichnet, dass** das Bispyridiniumalkan Octenidindihydrochlorid ist.

5. Zusammensetzung nach einem der vorherigen Ansprüche, **dadurch gekennzeichnet, dass** sie 0,01 bis 1 Gew.-% Bispyridiniumalkan enthält.

6. Zusammensetzung nach einem der vorhergehenden Ansprüche, **dadurch gekennzeichnet, dass** der aliphatische Alkohol Ethanol ist.

7. Nichttherapeutische Verwendung einer Zusammensetzung nach einem der vorherigen Ansprüche zur hygienischen Händedesinfektion oder zum desinfizierenden Händewaschen.

## Revendications

1. Composition alcoolique qui inclut
a) 0,02 à 2 % en poids d'un ou plusieurs 1- ou 2-(alkyle en C₁ à C₂₄)glycéroléthers choisis parmi le 1-(2-éthylhexyl)glycéroléther, le 1-dodécylglycéroléther et le 3-(1-méthoxy)-1,2-propanediol,
b) 0,01 à 2 % en poids d'un ou plusieurs bispyridiniumalcanes choisis parmi le (dichlorhydrate d'octénidine) et le disulfate d'octénidine,
c) 50 à 99 % en poids d'un ou plusieurs alcools aliphatiques choisis parmi l'éthanol, l'isopropanol et le n-propanol,
la composition comprenant également de 0,01 à 5 % en poids d'un ou plusieurs composés ammonium quaternaire choisis parmi l'étilsulfate de mécétronium et le chlorure de benzalkonium.

2. Composition selon la revendication 1, **caractérisée en ce que** le glycérolmonoalkyléther est le 1-(2-éthylhexyl)glycéroléther.

3. Composition selon l'une quelconque des revendications précédentes, **caractérisée en ce qu'**elle inclut 0,04 à 1 % en poids en poids de glycérolmonoalkyléther.

4. Composition selon l'une quelconque des revendications précédentes, **caractérisée en ce que** le bispyridiniumalcane est le dichlorhydrate d'octénidine.

5. Composition selon l'une quelconque des revendications précédentes, **caractérisée en ce qu'**elle inclut 0,01 à 1 % de bispyridiniumalcane.

6. Composition selon l'une quelconque des revendications précédentes, **caractérisée en ce que** l'alcool aliphatique est l'éthanol.

7. Utilisation non thérapeutique d'une composition selon l'une quelconque des revendications précédentes, pour une désinfection hygiénique des mains ou pour un lavage désinfectant des mains.
